# FASCICULE DE BREVET EUROPEEN

(11) **EP 4 572 826 B1**
(45) Date de publication et mention de la délivrance du brevet: **15.04.2026**
(21) Numéro de dépôt: 23768326.3
(22) Date de dépôt: 16.08.2023
(51) Int. Cl.: A61M 15/00

(54) **INHALATEUR DE POUDRE SÈCHE**
TROCKENPULVERINHALATOR
DRY-POWDER INHALER

(30) Priorité: 18.08.2022 FR 2208363
(43) Date de publication de la demande: 25.06.2025
(73) Titulaire: APTAR France SAS, 27110 Le Neubourg (FR)
(72) Inventeur: BELKHEROUF, Youcef, 27000 EVREUX (FR); MICHAUX, Sébastien, 76140 LE PETIT QUEVILLY (FR); TOURNAIRE, Jonathan, 76530 GRAND COURONNE (FR); POULLAIN, Franck, 27400 LA HAYE MALHERBE (FR); PETIT, Ludovic, 27110 VITOT (FR)
(74) Mandataire: Riege, Christian
(86) Numéro de dépôt international: PCT/FR2023/051269
(87) Numéro de publication internationale: WO 2024/038234

(56) Documents cités:
- WO-A1-03/075988
- WO-A1-2008/001132
- WO-A1-2013/008037
- WO-A2-2008/065403
- WO-A2-98/26828
- DE-A1- 102013 021 718
- US-A1- 2014 182 587

## Description

La présente invention concerne un inhalateur de poudre sèche.

Les inhalateurs de poudre sont bien connus dans l'état de la technique. Il en existe différentes sortes.

Un premier type d'inhalateur multidoses contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Ces dispositifs nécessitent des moyens de dosage complexes et donc coûteux, et la précision et la reproductibilité du dosage ne sont pas garanties. De plus, il y a des risques de contamination de la poudre disposée dans le réservoir au fur et à mesure de l'utilisation de l'inhalateur.

Un autre type d'inhalateur multidoses consiste à prévoir plusieurs réservoirs individuels contenant chacun une dose de poudre, puis d'ouvrir un de ces réservoirs à chaque actionnement de l'inhalateur. Cette mise en œuvre assure une meilleure étanchéité de la poudre, puisque chaque dose de poudre n'est exposée à l'atmosphère qu'au moment de son expulsion. Pour réaliser ces ensembles de réservoirs individuels, diverses variantes ont déjà été proposées, telle que des bandes ou disques de blisters. Concernant l'ouverture des réservoirs individuels, il a été proposé de peler ou décoller la couche de fermeture des blisters. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. Une autre solution est de percer la membrane de fermeture d'un blister à chaque actionnement, ce qui requiert des moyens de perçage complexes et donc coûteux.

Les inhalateurs multidoses décrits ci-dessus présentent de plus le problème de ne pas garantir une efficacité de la distribution optimale, avec une partie importante de la dose qui pénètre effectivement dans les poumons de l'utilisateur pour avoir un effet thérapeutique bénéfique.

Pour réaliser des dispositifs moins complexes et donc moins coûteux, il a été proposé des inhalateurs unidoses comportant un seul réservoir individuel, tels qu'un blister ou une capsule, qui est à charger dans l'inhalateur juste avant l'utilisation de celui-ci. L'avantage de ces dispositifs unidoses est qu'il n'est pas nécessaire de stocker l'ensemble des doses à l'intérieur de l'appareil, de sorte que celui-ci peut être de dimension réduite. Par contre, l'utilisation est plus compliquée pour l'utilisateur, puisque celui-ci est obligé de charger un blister ou une capsule dans l'inhalateur avant chaque utilisation. De plus, d'autres inconvénients spécifiques à ces inhalateurs unidoses existent. Ainsi, un blister ou une capsule ne permet pas la distribution de grandes doses, typiquement supérieures à 100 mg. De plus, l'ouverture, notamment des capsules ou blisters à percer, impose l'utilisation de moyens de perçage complexes, ainsi qu'un risque de résidus de membrane percée dans la poudre distribuée. Par ailleurs, les performances de vidage du réservoir ne sont pas toujours optimales, une partie de la poudre pouvant rester à l'intérieur du réservoir lors de l'inhalation.

Pour tenter de pallier à ces inconvénients, il a été proposé dans le document WO9826828 un inhalateur unidose comportant un réservoir cylindrique pourvu d'une ouverture latérale, disposé dans une chambre cylindrique de plus grand diamètre, de sorte que lors de l'inhalation, le réservoir se déplace dans la chambre selon un mouvement orbital, ce qui provoque l'expulsion de la poudre. Cette mise en œuvre permet effectivement d'améliorer l'efficacité de la distribution, avec un vidage sensiblement total du réservoir et une partie plus importante de la dose qui pénètre effectivement dans les poumons. Néanmoins, un inconvénient de ce dispositif est que l'ouverture latérale du réservoir doit être ouverte avant la mise en place du réservoir dans le dispositif, ce qui présente un risque de perte de poudre lors de cette mise en place, ou de contamination de celle-ci, notamment si le dispositif n'est pas utilisé rapidement après la mise en place du réservoir.

Les documents WO2008001132A1, WO03075988A1, US2014182587A1 et WO2013008037A1 décrivent d'autres dispositifs de l'état de la technique.

La présente invention a pour but de fournir un inhalateur de poudre qui ne reproduit pas les inconvénients susmentionnés.

En particulier, la présente invention a pour but de fournir un tel inhalateur qui améliore l'efficacité de distribution, en permettant la distribution de la quasi-totalité de la poudre contenue dans le réservoir et en augmentant la partie de la dose qui va atteindre les poumons de l'utilisateur.

La présente invention a également pour but de fournir un tel inhalateur qui limite voire empêche les risques de contamination et/ou de pollution de la poudre.

La présente invention a également pour but de fournir un tel inhalateur qui réduit la complexité d'utilisation pour l'utilisateur et qui garantit une meilleure sécurité d'utilisation.

La présente invention a aussi pour but de fournir un tel inhalateur qui soit simple et peu coûteux à fabriquer et à assembler.

La présente invention a donc pour objet un inhalateur de poudre sèche comportant :
- un corps contenant une chambre et un embout buccal pourvu d'un orifice de distribution à travers lequel l'utilisateur inhale,
- un réservoir contenant une dose de poudre sèche à inhaler et comportant un orifice latéral de sortie, ledit réservoir étant déplaçable entre une position non chargée, dans laquelle il est au moins partiellement disposé hors de ladite chambre, et une position chargée, dans laquelle il est entièrement disposé dans ladite chambre, le diamètre dudit réservoir étant inférieur au diamètre de ladite chambre, de sorte qu'en position chargée ledit réservoir peut se déplacer dans ladite chambre, ledit corps comportant des canaux tangentiels adaptés à amener un flux d'air d'inhalation de manière tangentielle dans ladite chambre, ce qui lors de l'inhalation génère un déplacement orbital dudit réservoir dans ladite chambre, en position chargée,
- un organe d'actionnement pour déplacer ledit réservoir de sa position non chargée vers sa position chargée,
ledit réservoir comportant un élément de fermeture indépendant dudit corps déplaçable et/ou déformable par rapport audit réservoir entre une position de fermeture dudit orifice latéral de sortie et une position d'ouverture dudit orifice latéral de sortie, ledit élément de fermeture étant en position de fermeture quand ledit réservoir est en position non chargée, et étant en position d'ouverture quand ledit réservoir est en position chargée.

Avantageusement, ledit élément de fermeture comporte une projection radiale asymétrique.

Avantageusement, ledit élément de fermeture comporte au moins une seconde projection radiale qui s'étend axialement sur une partie de la hauteur axiale dudit élément de fermeture.

Avantageusement, ledit réservoir est formé d'une partie supérieure de réservoir et d'une partie inférieure de réservoir, qui sont assemblées après remplissage avec la dose de poudre.

Avantageusement, ladite partie inférieure de réservoir comporte ledit orifice latéral de sortie.

La présente invention a également pour objet un inhalateur de poudre sèche comportant :
- un corps contenant une chambre et un embout buccal pourvu d'un orifice de distribution à travers lequel l'utilisateur inhale,
- un réservoir contenant une dose de poudre sèche à inhaler et comportant un orifice latéral de sortie, ledit réservoir étant déplaçable entre une position non chargée, dans laquelle il est au moins partiellement disposé hors de ladite chambre, et une position chargée, dans laquelle il est entièrement disposé dans ladite chambre, le diamètre dudit réservoir étant inférieur au diamètre de ladite chambre, de sorte qu'en position chargée ledit réservoir peut se déplacer dans ladite chambre, ledit corps comportant des canaux tangentiels adaptés à amener un flux d'air d'inhalation de manière tangentielle dans ladite chambre, ce qui lors de l'inhalation génère un déplacement orbital dudit réservoir dans ladite chambre, en position chargée,
- un organe d'actionnement pour déplacer ledit réservoir de sa position non chargée vers sa position chargée,
ledit réservoir comportant une partie supérieure de réservoir pourvue d'un orifice latéral supérieur et une partie inférieure de réservoir pourvue d'un orifice latéral inférieur, ladite partie supérieure de réservoir étant déplaçable axialement par rapport à ladite partie inférieure de réservoir, lesdits orifices latéraux supérieur et inférieur étant décalés axialement quand ledit réservoir est en position non chargée, et étant alignés pour former un orifice latéral ouvert quand ledit réservoir est en position chargée.

Avantageusement, ledit corps comporte deux parties de corps, une partie de corps supérieure comportant ledit embout buccal et une partie de corps inférieure comportant un manchon cylindrique, ladite chambre étant définie par ces deux parties de corps fixées l'une à l'autre.

Avantageusement, ledit embout buccal comporte une grille en amont dudit orifice de distribution.

Avantageusement, ladite chambre est disposée entre une entrée d'air d'inhalation et ledit embout buccal.

Avantageusement, ledit réservoir contient une dose de poudre supérieure à 50 mg, notamment supérieure à 100 mg.

Avantageusement, le volume occupé par ledit réservoir dans ladite chambre en position chargée est supérieur à 50% du volume de ladite chambre.

Avantageusement, le diamètre radial dudit réservoir est supérieur à sa hauteur axiale.

Avantageusement, ledit organe d'actionnement est déplaçable axialement par rapport audit corps pour déplacer ledit réservoir de sa position non chargée vers sa position chargée.

Avantageusement, ledit organe d'actionnement comporte au moins une rainure oblique recevant au moins un ergot respectif formé sur ledit corps ou sur un élément solidaire dudit corps, de sorte que lors de son déplacement axial, ledit organe d'actionnement réalise une rotation dans ledit corps.

Ces caractéristiques et avantages et d'autres de la présente invention apparaîtront plus clairement au cours de la description détaillée suivante, faite en référence aux dessins joints, donnés à titre d'exemples non limitatifs, sur lesquels
la figure 1 est une vue schématique en section transversale d'un inhalateur selon un premier mode de réalisation avantageux de l'invention, en position non chargée,
la figure 2 est une vue similaire à celle de la figure 1, en position chargée,
la figure 3 est une vue schématique partielle en section transversale selon un autre plan de coupe du dispositif de distribution de la figure 1, en position non chargée,
la figure 4 est une vue similaire à celle de la figure 3, en position chargée,
la figure 5 est une vue schématique de dessous du réservoir dans la chambre, en position chargée,
la figure 6 est une vue schématique de dessus de l'embout buccal,
la figure 7 est une vue schématique en section verticale du réservoir, en position non chargée,
la figure 8 est une vue similaire à celle de la figure 7, en position chargée,
la figure 9 est une vue schématique similaire à celle de la figure 7, selon un autre angle de vue, en position non chargée,
la figure 10 est une vue similaire à celle de la figure 9, en position chargée,
les figures 11 à 13 sont des vues schématiques en section verticale de trois variantes de réalisation avantageuses du réservoir, en position non-chargée,
les figures 14 à 16 sont des vues schématiques en section verticale d'un réservoir selon un autre mode de réalisation, respectivement avant assemblage, en position non chargée et en position chargée,
la figure 17 est une vue schématique en perspective montrant le réservoir selon encore un autre mode de réalisation, en position chargée,
les figures 18 et 19 sont des vues schématiques en section verticale du réservoir de la figure 17, respectivement en position non chargée et en position chargée,
la figure 20 est une vue schématique en perspective d'un élément de fermeture selon une autre variante avantageuse,
la figure 21 illustre l'assemblage possible dans la bonne orientation de l'élément de fermeture de la figure 21, et
la figure 22 illustre l'assemblage impossible dans la mauvaise orientation de l'élément de fermeture de la figure 21.

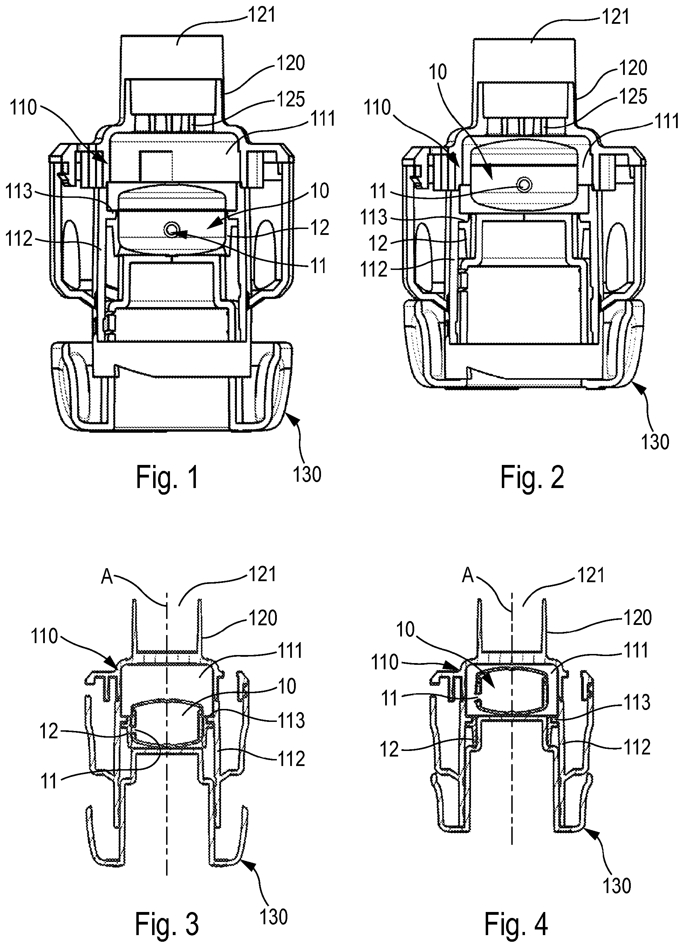

Dans la description ci-après, les termes "supérieur", "inférieur" et "latéral" se réfèrent à la position droite du dispositif représenté sur les figures 1 à 4. Les termes "axial" et "radial" se réfèrent à l'axe central longitudinal A du dispositif, représenté sur les figures 3 et 4.

Les figures 1 à 10 décrivent un premier mode de réalisation de l'invention.

Dans ce premier mode de réalisation, l'inhalateur comporte un corps 110 contenant une chambre 111 et un embout buccal 120 pourvu d'un orifice de distribution 121 à travers lequel l'utilisateur inhale.

Avantageusement, le corps 110 est formé de deux parties de corps, une partie de corps supérieure comportant l'embout buccal 120 et une partie de corps inférieure comportant un manchon cylindrique 112, la chambre 111 étant définie par ces deux parties de corps fixées l'une à l'autre.

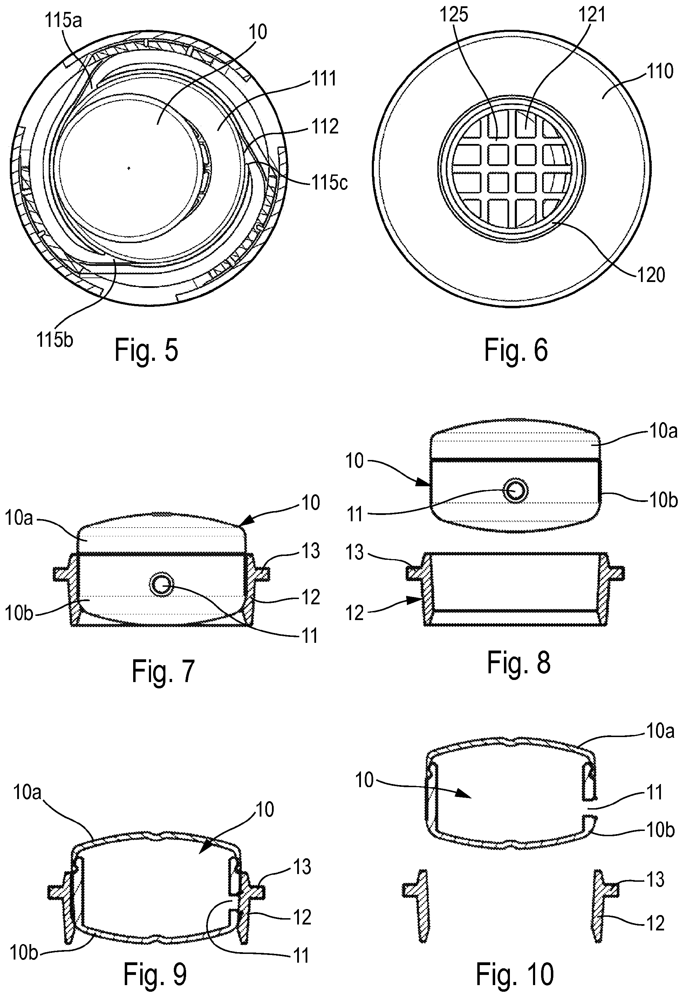

Avantageusement, comme visible sur la figure 6, l'embout buccal 120 comporte une grille 125 en amont de l'orifice de distribution 121.

De préférence, la chambre 111 est disposée entre une entrée d'air d'inhalation et ledit embout buccal 120, de sorte que lorsque l'utilisateur inhale à travers l'embout buccal 120, le flux d'air va traverser ladite chambre 111.

L'inhalateur comporte un réservoir 10 contenant une dose de poudre sèche à inhaler et comportant un orifice latéral de sortie 11. Ce réservoir 10 est déplaçable entre une position non chargée, dans laquelle il est au moins partiellement disposé hors de ladite chambre 111, et une position chargée, dans laquelle il est entièrement disposé dans ladite chambre 111.

Le diamètre du réservoir 10 est inférieur au diamètre de ladite chambre 111, de sorte qu'en position chargée, le réservoir 10 peut se déplacer dans la chambre 111. Des canaux tangentiels 115a, 115b, 115c amènent ledit flux d'air d'inhalation de manière tangentielle dans la chambre 111, ce qui va provoquer un déplacement orbital du réservoir 10 dans la chambre 111, le réservoir 10 tournant sur lui-même tout en tournant autour de la périphérie de la chambre 111 le long de la paroi latérale de celle-ci. Ce mouvement va permettre l'expulsion de la poudre contenue dans le réservoir 10, qui est entrainée par le flux d'air d'inhalation vers l'embout buccal et l'orifice de distribution.

Avantageusement, le réservoir 10 peut contenir une dose de poudre supérieure à 50 mg, notamment supérieure à 100 mg. Eventuellement, des doses très importantes peuvent être envisagées, notamment supérieures à 500 mg.

Avantageusement, le volume occupé par le réservoir 10 dans la chambre 111 en position chargée est supérieur à 50% du volume de la chambre 111.

Avantageusement, le diamètre radial du réservoir 10 est supérieur à sa hauteur axiale.

Avantageusement, le réservoir 10 est formé d'une partie supérieure de réservoir 10a et d'une partie inférieure de réservoir 10b, qui sont assemblées après remplissage avec la dose de poudre.

L'inhalateur comporte aussi un organe d'actionnement 130 pour déplacer ledit réservoir 10 de sa position non chargée vers sa position chargée. Cet organe d'actionnement 130 est déplaçable axialement par rapport au corps 110 pour pousser le réservoir 10 de sa position non chargée vers sa position chargée.

Avantageusement, le déplacement axial de l'organe d'actionnement 130 se fait par une rotation de l'organe d'actionnement 130 dans le corps 110, par exemple au moyen d'ergot(s) du corps 110 disposé(s) dans une(des) rainure(s) oblique(s) de l'organe d'actionnement 130.

Selon le premier mode de réalisation de l'invention représenté sur les figures 1 à 10, le réservoir 10 comporte un orifice latéral de sortie 11 obturé par un élément de fermeture 12 lorsque le réservoir 10 n'est pas dans sa position chargée.

Avantageusement, c'est la partie inférieure de réservoir 10b qui comporte l'orifice latéral de sortie 11.

Cet élément de fermeture 12 protège donc la poudre contenue dans le réservoir jusqu'à sa distribution par l'actionnement de l'inhalateur.

Ainsi, selon un premier aspect de l'invention, l'élément de fermeture 12 est déplaçable et/ou déformable par rapport au réservoir 10 entre des positions de fermeture et d'ouverture de l'orifice latéral de sortie 11, ledit élément de fermeture 12 étant en position de fermeture quand ledit réservoir 10 est en position non chargée, et étant en position d'ouverture quand ledit réservoir 10 est en position chargée.

Avantageusement, en position non chargée, l'élément de fermeture 12 est en butée sur une partie du corps 110 ou solidaire de celui-ci, de sorte que lorsque le réservoir 10 est déplacé vers sa position chargée, l'élément de fermeture 12 reste bloqué dans le corps, ouvrant ainsi l'orifice latéral de sortie 11.

Dans l'exemple des figures 1 à 10, l'élément de fermeture 12 comporte une projection radiale 13 qui coopère avec un épaulement 113 du manchon cylindrique 112.

Avantageusement, la projection radiale 13 est disposée d'un côté de l'élément de fermeture 12, de sorte que celui-ci n'est pas symétrique. Ainsi, lorsque l'élément de fermeture 12 est en position de fermeture sur le réservoir 10, celui-ci n'est pas non plus symétrique, ce qui forme un détrompage pour l'insertion du réservoir 10 dans le corps 110 de l'inhalateur, cette insertion n'étant possible que dans une seule orientation du réservoir 10. Ceci est notamment avantageux lorsque la forme extérieure du réservoir 10 est sensiblement symétrique mais que l'orifice latéral de sortie 11 n'est pas disposée au centre axial du réservoir 10, comme dans les exemples des figures. En effet, dans ce cas, il est souhaitable d'orienter correctement le réservoir 10 dans l'inhalateur pour un fonctionnement optimal, et la forme asymétrique de l'élément de fermeture permet de remplir cette fonction.

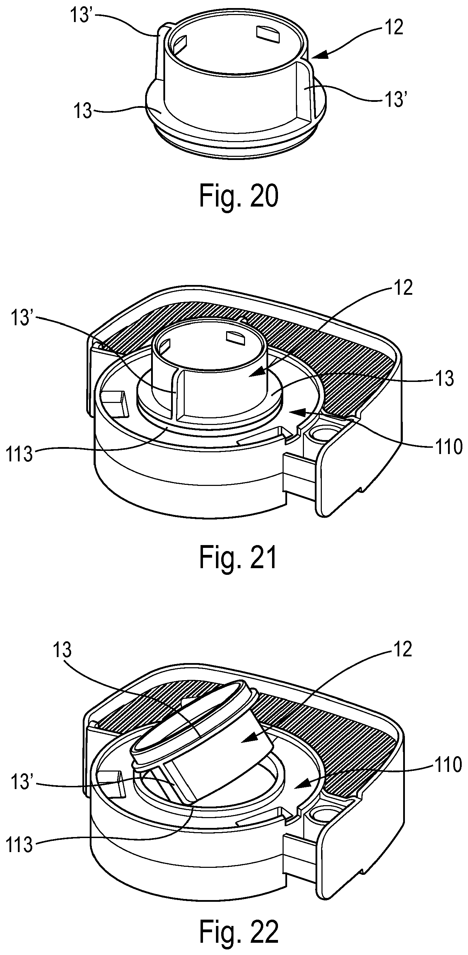

Les figures 20 à 22 montrent une autre variante dans laquelle l'élément de fermeture 12 comporte au moins une seconde projection radiale 13' qui s'étend axialement sur une partie de la hauteur de l'élément de fermeture 12, avantageusement entre un bord axial et ladite projection radiale 13. Ceci permet d'éviter tout risque d'assembler le réservoir 10 à l'envers, comme illustré sur les figures 21 et 22. Dans la figure 21, l'orientation est correcte, et l'ensemble réservoir 10 + élément de fermeture 12 peut être assemblé dans le corps 110, alors que dans l'orientation de la figure 22, cet assemblage est rendu impossible par la présence de la seconde projection radiale 13'. Dans l'exemple représenté, il y a deux secondes projections radiales 13' diamétralement opposées, mais un nombre quelconque est possible.

Bien entendu, si le réservoir 10 est symétrique, avec l'orifice latéral de sortie 11 centré axialement, l'élément de fermeture 12 n'a pas besoin d'être asymétrique. Au contraire, un élément de fermeture 12 formant avec le réservoir 10 une unité symétrique permettrait à l'utilisateur d'insérer cette unité dans l'inhalateur dans les deux orientations, sans risques de dysfonctionnement ou distribution de poudre moins efficace.

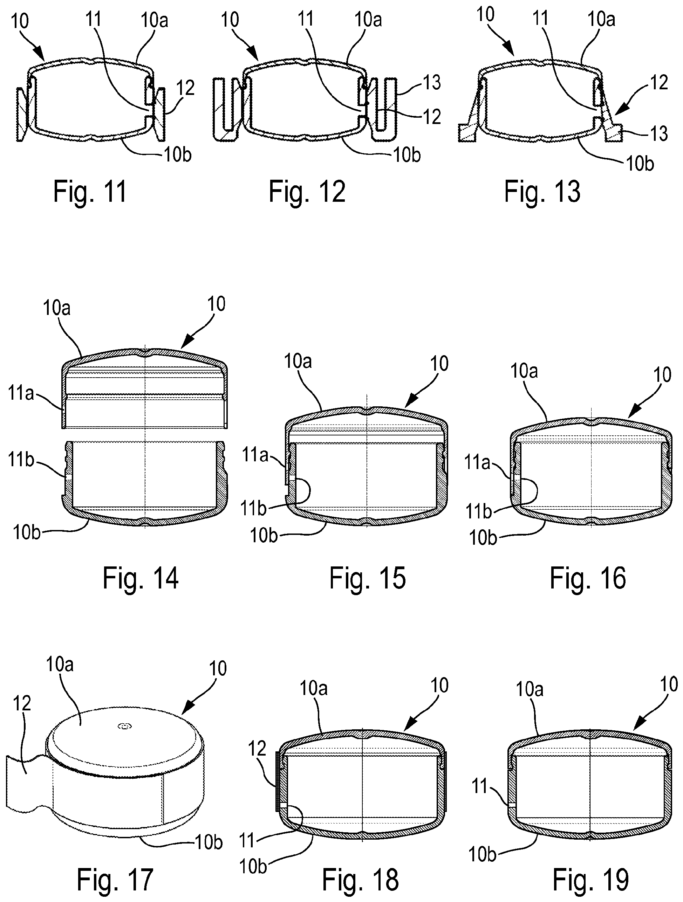

Des variantes de réalisation de l'élément de fermeture 12 sont représentées sur les figures 11 à 13.

Les figures 14 à 16 illustrent un second mode de réalisation. Ici, l'élément de fermeture est formé directement par la partie supérieure de réservoir 10a, et il n'est pas nécessaire de prévoir un élément de fermeture séparé.

Dans ce second mode de réalisation, la partie supérieure de réservoir 10a est pourvue d'un orifice latéral supérieur 11a et la partie inférieure de réservoir 10b est pourvue d'un orifice latéral inférieur 11b. La partie supérieure de réservoir 10a est déplaçable axialement par rapport à ladite partie inférieure de réservoir 10b, avec lesdits orifices latéraux supérieur et inférieur 11a, 11b qui sont décalés axialement quand ledit réservoir 10 est en position non chargée, et qui sont alignés pour former un orifice latéral ouvert quand ledit réservoir 10 est en position chargée.

Les figures 17 à 19 illustrent un troisième mode de réalisation. Ici, l'élément de fermeture 12 est formé par une bande souple fixée ou collée autour du réservoir 10 pour fermer l'orifice latéral de sortie 11. L'inhalateur comporte dans ce cas des moyens de fixation de l'extrémité libre de ladite bande souple, de sorte que lorsque le réservoir 10 est déplacé de sa position non chargée vers sa position chargée, l'élément de fermeture 12 est retiré du réservoir 10. Par exemple, on peut réaliser une rotation du réservoir 10, ensemble avec celle de l'organe d'actionnement 130, pour décoller la bande souple. En variante, on peut prévoir un ressort préchargé relié à ladite bande souple, et qui est libéré pour tirer sur ladite bande souple lorsque le réservoir est déplacé axialement vers sa position chargée.

Le dispositif de l'invention est simple et efficace. Il est constitué d'un faible nombre de pièces, il est donc peu coûteux à fabriquer et à assembler, et fiable d'utilisation. Il permet une distribution optimale de la poudre de par le mouvement orbital du réservoir lors de l'actionnement, tout en garantissant l'intégrité de la poudre jusqu'à son utilisation.

Il est à noter que l'inhalateur peut être rechargeable, en ouvrant le corps 110 pour retirer le réservoir vide et le remplacer par un réservoir plein.

Diverses modifications sont également possibles pour un homme du métier sans sortir du cadre de la présente invention tel que défini par les revendications annexées.

## Revendications

1. Inhalateur de poudre sèche comportant :
- un corps (110) contenant une chambre (111) et un embout buccal (120) pourvu d'un orifice de distribution (121) à travers lequel l'utilisateur inhale,
- un réservoir (10) contenant une dose de poudre sèche à inhaler et comportant un orifice latéral de sortie (11), ledit réservoir (10) étant déplaçable entre une position non chargée, dans laquelle il est au moins partiellement disposé hors de ladite chambre (111), et une position chargée, dans laquelle il est entièrement disposé dans ladite chambre (111), le diamètre dudit réservoir (10) étant inférieur au diamètre de ladite chambre (111), de sorte qu'en position chargée ledit réservoir (10) peut se déplacer dans ladite chambre (111), ledit corps (110) comportant des canaux tangentiels (115a, 115b, 115c) adaptés à amener un flux d'air d'inhalation de manière tangentielle dans ladite chambre (111), ce qui lors de l'inhalation génère un déplacement orbital dudit réservoir (10) dans ladite chambre (111), en position chargée,
- un organe d'actionnement (130) pour déplacer ledit réservoir (10) de sa position non chargée vers sa position chargée,
**caractérisé en ce que** ledit réservoir (10) comporte un élément de fermeture (12) indépendant dudit corps (110) déplaçable et/ou déformable par rapport audit réservoir (10) entre une position de fermeture dudit orifice latéral de sortie (11) et une position d'ouverture dudit orifice latéral de sortie (11), ledit élément de fermeture (12) étant en position de fermeture quand ledit réservoir (10) est en position non chargée, et étant en position d'ouverture quand ledit réservoir (10) est en position chargée.

2. Inhalateur selon la revendication 1, dans lequel ledit élément de fermeture (12) comporte une projection radiale asymétrique (13).

3. Inhalateur selon la revendication 2, dans lequel ledit élément de fermeture (12) comporte au moins une seconde projection radiale (13') qui s'étend axialement sur une partie de la hauteur axiale dudit élément de fermeture (12).

4. Inhalateur selon l'une quelconque des revendications précédentes, dans lequel ledit réservoir (10) est formé d'une partie supérieure de réservoir (10a) et d'une partie inférieure de réservoir (10b), qui sont assemblées après remplissage avec la dose de poudre.

5. Inhalateur selon la revendication 4, dans lequel ladite partie inférieure de réservoir (10b) comporte ledit orifice latéral de sortie (11).

6. Inhalateur de poudre sèche comportant :
- un corps (110) contenant une chambre (111) et un embout buccal (120) pourvu d'un orifice de distribution (121) à travers lequel l'utilisateur inhale,
- un réservoir (10) contenant une dose de poudre sèche à inhaler et comportant un orifice latéral de sortie (11a, 11b), ledit réservoir (10) étant déplaçable entre une position non chargée, dans laquelle il est au moins partiellement disposé hors de ladite chambre (111), et une position chargée, dans laquelle il est entièrement disposé dans ladite chambre (111), le diamètre dudit réservoir (10) étant inférieur au diamètre de ladite chambre (111), de sorte qu'en position chargée ledit réservoir (10) peut se déplacer dans ladite chambre (111), ledit corps (110) comportant des canaux tangentiels (115a, 115b, 115c) adaptés à amener un flux d'air d'inhalation de manière tangentielle dans ladite chambre (111), ce qui lors de l'inhalation génère un déplacement orbital dudit réservoir (10) dans ladite chambre (111), en position chargée,
- un organe d'actionnement (130) pour déplacer ledit réservoir (10) de sa position non chargée vers sa position chargée,
**caractérisé en ce que** ledit réservoir (10) comporte une partie supérieure de réservoir (10a) pourvue d'un orifice latéral supérieur (11a) et une partie inférieure de réservoir (10b) pourvue d'un orifice latéral inférieur (11b), ladite partie supérieure de réservoir (10a) étant déplaçable axialement par rapport à ladite partie inférieure de réservoir (10b), lesdits orifices latéraux supérieur et inférieur (11a, 11b) étant décalés axialement quand ledit réservoir (10) est en position non chargée, et étant alignés pour former un orifice latéral ouvert quand ledit réservoir (10) est en position chargée.

7. Inhalateur selon l'une quelconque des revendications précédentes, dans lequel ledit corps (110) comporte deux parties de corps, une partie de corps supérieure comportant ledit embout buccal (120) et une partie de corps inférieure comportant un manchon cylindrique (112), ladite chambre (111) étant définie par ces deux parties de corps fixées l'une à l'autre.

8. Inhalateur selon l'une quelconque des revendications précédentes, dans lequel ledit embout buccal (120) comporte une grille (125) en amont dudit orifice de distribution (121).

9. Inhalateur selon l'une quelconque des revendications précédentes, dans lequel ladite chambre (111) est disposée entre une entrée d'air d'inhalation et ledit embout buccal (120).

10. Inhalateur selon l'une quelconque des revendications précédentes, dans lequel ledit réservoir (10) contient une dose de poudre supérieure à 50 mg, notamment supérieure à 100 mg.

11. Inhalateur selon l'une quelconque des revendications précédentes, dans lequel le volume occupé par ledit réservoir (10) dans ladite chambre (111) en position chargée est supérieur à 50% du volume de ladite chambre (111).

12. Inhalateur selon l'une quelconque des revendications précédentes, dans lequel le diamètre radial dudit réservoir (10) est supérieur à sa hauteur axiale.

13. Inhalateur selon l'une quelconque des revendications précédentes, dans lequel ledit organe d'actionnement (130) est déplaçable axialement par rapport audit corps (110) pour déplacer ledit réservoir (10) de sa position non chargée vers sa position chargée.

14. Inhalateur selon la revendication 13, dans lequel ledit organe d'actionnement (130) comporte au moins une rainure oblique recevant au moins un ergot respectif formé sur ledit corps (110) ou sur un élément solidaire dudit corps (110), de sorte que lors de son déplacement axial, ledit organe d'actionnement (130) réalise une rotation dans ledit corps (110).

## Patentansprüche

1. Trockenpulverinhalator, umfassend:
- einen Körper (110), der eine Kammer (111) und ein Mundstück (120) umfasst, das mit einer Verteilungsöffnung (121) versehen ist, durch die der Benutzer inhaliert,
- einen Behälter (10), der eine zu inhalierende Trockenpulverdosis enthält und eine seitliche Austrittsöffnung (11) umfasst, wobei der Behälter (10) zwischen einer nicht geladenen Position, in der er zumindest teilweise außerhalb der Kammer (111) angeordnet ist, und einer geladenen Position verlagerbar ist, in der er vollständig in der Kammer (111) angeordnet ist, wobei der Durchmesser des Behälters (10) kleiner als der Durchmesser der Kammer (111) ist, derart dass der Behälter (10) sich in der geladenen Position in der Kammer (111) verlagern kann, wobei der Körper (110) tangentiale Kanäle (115a, 115b, 115c) umfasst, die dazu geeignet sind, eine Inhalationsluftströmung tangential in die Kammer (111) zu führen, wodurch bei der Inhalation eine orbitale Verlagerung des Behälters (10) in der geladenen Position in der Kammer (111) erzeugt wird,
- ein Betätigungsorgan (130) zum Verlagern des Behälters (10) von seiner nicht geladenen Position hin zu seiner geladenen Position,
**dadurch gekennzeichnet, dass** der Behälter (10) ein von dem Körper (110) unabhängiges Schließelement (12) umfasst, das in Bezug auf den Behälter (10) zwischen einer Schließposition der seitlichen Austrittsöffnung (11) und einer Öffnungsposition der seitlichen Austrittsöffnung (11) verlagerbar und/oder verformbar ist, wobei das Schließelement (12) sich in der Schließposition befindet, wenn der Behälter (10) sich in der nicht geladenen Position befindet, und sich in der Öffnungsposition befindet, wenn der Behälter (10) sich in der geladenen Position befindet.

2. Inhalator nach Anspruch 1, wobei das Schließelement (12) eine asymmetrische radiale Projektion (13) umfasst.

3. Inhalator nach Anspruch 2, wobei das Schließelement (12) mindestens eine zweite radiale Projektion (13') umfasst, die sich axial auf einem Teil der axialen Höhe des Schließelements (12) erstreckt.

4. Inhalator nach einem der vorhergehenden Ansprüche, wobei der Behälter (10) aus einem oberen Behälterteil (10a) und einem unteren Behälterteil (10b) gebildet ist, die nach dem Füllen mit der Pulverdosis zusammengebaut werden.

5. Inhalator nach Anspruch 4, wobei der untere Behälterteil (10b) die seitliche Austrittsöffnung (11) umfasst.

6. Trockenpulverinhalator, umfassend:
- einen Körper (110), der eine Kammer (111) und ein Mundstück (120) umfasst, das mit einer Verteilungsöffnung (121) versehen ist, durch die der Benutzer inhaliert,
- einen Behälter (10), der eine zu inhalierende Trockenpulverdosis enthält und eine seitliche Austrittsöffnung (11a, 11b) umfasst, wobei der Behälter (10) zwischen einer nicht geladenen Position, in der er zumindest teilweise außerhalb der Kammer (111) angeordnet ist, und einer geladenen Position verlagerbar ist, in der er vollständig in der Kammer (111) angeordnet ist, wobei der Durchmesser des Behälters (10) kleiner als der Durchmesser der Kammer (111) ist, derart dass der Behälter (10) sich in der geladenen Position in der Kammer (111) verlagern kann, wobei der Körper (110) tangentiale Kanäle (115a, 115b, 115c) umfasst, die dazu geeignet sind, eine Inhalationsluftströmung tangential in die Kammer (111) zu führen, wodurch bei der Inhalation eine orbitale Verlagerung des Behälters (10) in der geladenen Position in der Kammer (111) erzeugt wird,
- ein Betätigungsorgan (130) zum Verlagern des Behälters (10) von seiner nicht geladenen Position hin zu seiner geladenen Position,
**dadurch gekennzeichnet, dass** der Behälter (10) einen oberen Behälterteil (10a), der mit einer oberen seitlichen Öffnung (11a) versehen ist, und einen unteren Behälterteil (10b) umfasst, der mit einer unteren seitlichen Öffnung (11b) versehen ist, wobei der obere Behälterteil (10a) axial in Bezug auf den unteren Behälterteil (10b) verlagerbar ist, die obere und die untere seitliche Öffnung (11a, 11b) axial versetzt sind, wenn der Behälter (10) sich in der nicht geladenen Position befindet, und ausgerichtet sind, um eine offene seitliche Öffnung zu bilden, wenn der Behälter (10) sich in der geladenen Position befindet.

7. Inhalator nach einem der vorhergehenden Ansprüche, wobei der Körper (110) zwei Körperteile umfasst, einen oberen Körperteil, der das Mundstück (120) umfasst, und einen unteren Körperteil, der eine zylindrische Hülse (112) umfasst, wobei die Kammer (111) durch diese zwei aneinander befestigten Körperteile definiert ist.

8. Inhalator nach einem der vorhergehenden Ansprüche, wobei das Mundstück (120) ein Gitter (125) stromaufwärts der Verteilungsöffnung (121) umfasst.

9. Inhalator nach einem der vorhergehenden Ansprüche, wobei die Kammer (111) zwischen einem Inhalationslufteinlass und dem Mundstück (120) angeordnet ist.

10. Inhalator nach einem der vorhergehenden Ansprüche, wobei der Behälter (10) eine Pulverdosis von größer als 50 mg, insbesondere größer als 100 mg, enthält.

11. Inhalator nach einem der vorhergehenden Ansprüche, wobei das von dem Behälter (10) in der Kammer (111) in der geladenen Position eingenommene Volumen größer als 50 % des Volumens der Kammer (111) ist.

12. Inhalator nach einem der vorhergehenden Ansprüche, wobei der radiale Durchmesser des Behälters (10) größer als seine axiale Höhe ist.

13. Inhalator nach einem der vorhergehenden Ansprüche, wobei das Betätigungsorgan (130) axial in Bezug auf den Körper (110) verlagerbar ist, um den Behälter (10) von seiner nicht geladenen Position hin zu seiner geladenen Position zu verlagern.

14. Inhalator nach Anspruch 13, wobei das Betätigungsorgan (130) mindestens eine schräge Nut umfasst, die mindestens einen jeweiligen Stift aufnimmt, der auf dem Körper (110) oder einem fest mit dem Körper (110) verbundenen Element gebildet ist, derart dass das Betätigungsorgan (130) bei seiner axialen Verlagerung eine Drehung in dem Körper (110) ausführt.

## Claims

1. A dry-powder inhaler comprising:
- a body (110) containing a chamber (111) and a mouthpiece (120) provided with a dispensing orifice (121) through which the user inhales,
- a reservoir (10) containing a dose of dry powder to be inhaled and comprising a lateral outlet opening (11), said reservoir (10) being movable between an non-loaded position, in which it is at least partially disposed outside said chamber (111), and a loaded position, in which it is entirely disposed in said chamber (111), the diameter of said reservoir (10) being less than the diameter of said chamber (111), so that, in the loaded position, said reservoir (10) can move in said chamber (111), said body (110) comprising tangential channels (115a, 115b, 115c) adapted to bring a flow of inhalation air tangentially into said chamber (111), which, during inhalation, generates an orbital movement of said reservoir (10) in said chamber (111), in the loaded position,
- an actuating member (130) for moving said reservoir (10) from its non-loaded position towards its loaded position,
**characterized in that** said reservoir (10) includes a closure element (12) which is independent from said body (110) displaceable and/or deformable with respect to said reservoir (10) between a closed position of said lateral outlet opening (11)and an open position of said lateral outlet opening (11), said closure element (12) being in the closed position when said reservoir (10) is in the non-loaded position, and being in the open position when said reservoir (10) is in the loaded position.

2. The inhaler according to claim 1, wherein said closure element (12) comprises an asymmetrical radial projection (13).

3. The inhaler according to claim 2, wherein said closure element (12) comprises at least one second radial projection (13') which extends axially over part of the axial height of said closure element (12).

4. The inhaler as claimed in any one of the preceding claims, wherein said reservoir (10) is formed by an upper reservoir portion (10a) and a lower reservoir portion (10b), which are assembled after filling with the dose of powder.

5. The inhaler according to claim 4, wherein said lower reservoir portion (10b) comprises said lateral outlet opening (11).

6. A dry-powder inhaler comprising:
- a body (110) containing a chamber (111) and a mouthpiece (120) provided with a dispensing orifice (121) through which the user inhales,
- a reservoir (10) containing a dose of dry powder to be inhaled and comprising a lateral outlet opening (11 a, 11 b), said reservoir (10) being movable between an non-loaded position, in which it is at least partially disposed outside said chamber (111), and a loaded position, in which it is entirely disposed in said chamber (111), the diameter of said reservoir (10) being less than the diameter of said chamber (111), so that, in the loaded position, said reservoir (10) can move in said chamber (111), said body (110) comprising tangential channels (115a, 115b, 115c) adapted to bring a flow of inhalation air tangentially into said chamber (111), which, during inhalation, generates an orbital movement of said reservoir (10) in said chamber (111), in the loaded position,
- an actuating member (130) for moving said reservoir (10) from its non-loaded position towards its loaded position,
**characterized in that** said reservoir (10) comprises an upper reservoir portion (10a) provided with an upper lateral opening (11a) and a lower reservoir portion (10b) provided with a lower lateral opening (11b), said upper reservoir portion (10a) being axially movable relative to said lower reservoir portion (10b), said upper and lower lateral openings (11a, 11b) being axially offset when said reservoir (10) is in the non-loaded position, and being aligned to form an open lateral opening when said reservoir (10) is in the loaded position.

7. The inhaler as claimed in any one of the preceding claims, wherein said body (110) comprises two body portions, an upper body portion comprising said mouthpiece (120) and a lower body portion comprising a cylindrical sleeve (112), said chamber (111) being defined by these two body portions fixed to one another.

8. The inhaler as claimed in any one of the preceding claims, wherein said mouthpiece (120) comprises a grid (125) upstream from said dispensing orifice (121).

9. The inhaler as claimed in any one of the preceding claims, wherein said chamber (111) is disposed between an inhalation air inlet and said mouthpiece (120).

10. The inhaler as claimed in any one of the preceding claims, wherein said reservoir (10) contains a dose of powder greater than 50 mg, in particular greater than 100 mg.

11. The inhaler as claimed in any one of the preceding claims, wherein the volume occupied by said reservoir (10) in said chamber (111) in the loaded position is greater than 50% of the volume of said chamber (111).

12. The inhaler as claimed in any one of the preceding claims, wherein the radial diameter of said reservoir (10) is greater than its axial height.

13. The inhaler as claimed in any one of the preceding claims, in which said actuating member (130) is axially displaceable with respect to said body (110) in order to displace said reservoir (10) from its non-loaded position towards its loaded position.

14. The inhaler according to claim 13, wherein said actuating member (130) comprises at least one oblique groove receiving at least one respective lug formed on said body (110) or on an element which is secured to said body (110), in a manner such that during its axial displacement, said actuating member (130) implements a rotation in said body (110).
